# EUROPEAN PATENT SPECIFICATION

(11) **EP 1 140 228 B1**
(45) Date of publication and mention of the grant of the patent: **10.03.2004**
(21) Application number: 99964875.1
(22) Date of filing: 15.12.1999
(51) Int. Cl.: A61L 15/46, A61L 15/60

(54) **AN ABSORBENT ARTICLE HAVING A MATERIAL LAMINATE THAT COMPRISES A LIQUID PERMEABLE TOP SHEET AND A LIQUID PERMEABLE LIQUID TRANSFER SHEET**
ABSORBIERENDER ARTIKEL MIT EINEM MEHRSCHICHTMATERIAL, DAS EINE FLÜSSIGKEITSDURCHLÄSSIGE OBERSCHICHT UND EINE FLÜSSIGKEITSDURCHLÄSSIGE FLÜSSIGKEITSTRANSFERSCHICHT ENTHÄLT
ARTICLE ABSORBANT A MATERIAU LAMINE COMPRENANT UNE FEUILLE SUPERIEURE PERMEABLE AUX LIQUIDES ET UNE FEUILLE DE TRANSFERT DE LIQUIDE PERMEABLE AUX LIQUIDES

(30) Priority: 16.12.1998 SE 9804360
(43) Date of publication of application: 10.10.2001
(73) Proprietor: SCA Hygiene Products AB, 405 03 Göteborg (SE)
(72) Inventor: HAGRUD, Ulrika, S-413 22 Göteborg (SE)
(74) Representative: Larsson, Karin
(86) International application number: PCT/SE1999/002370
(87) International publication number: WO 2000/035503

(56) References cited:
- EP-A2- 0 202 126
- EP-A2- 0 391 108
- DE-A1- 19 512 005
- US-A- 5 522 811

## Description

The present invention relates to an absorbent article that comprises an absorbent body enclosed between a liquid impermeable backing sheet and a material laminate in the form of a liquid permeable outer sheet or top sheet and a liquid permeable liquid transfer sheet, with the liquid permeable transfer sheet lying proximal to the absorbent body.

### BACKGROUND

A common problem encountered with the absorbent articles such as diapers, sanitary napkins, incontinence protectors and the like is that their use can lead to undesired side effects, such as skin irritation and problems associated with body waste odours. These problems can arise as a result of occlusion, the presence of moisture, and of mechanical, microbial and enzymatic factors, all of which coact mutually to different extents and amplify the effect of one another. Several undesired side effects can also arise as a result of or in conjunction with an increase in pH.

U.S. 3,794,034 describes the significance of pH in an absorbent article and teaches impregnation of the article with buffering substances that enable the pH in the article to be kept between 3.5 and 6.0, which is beneficial with respect to both inhibiting the growth of undesired bacteria and therewith the occurrence of undesired odours, and also in avoiding a negative effect on the wearer's skin.

Swedish Patent Application SE 9702298-2 teaches the use of an absorbent article that includes a pH-regulating substance in the form of a partially neutralised superabsorbent material where, after wetting, the pH in the article will lie between 3.5 and 4.9. An absorbent article according to SE 9702298-2 reduces the risk of skin irritation and also problems associated with bad odours. A conventional superabsorbent material has a degree of neutralisation of about 70%, whereas the partially neutralised superabsorbent material has a lower degree of neutralisation.

### SUMMARY OF THE INVENTION

The object of the present invention is to reduce the risk of skin irritation still further, such as contact dermatitis for instance. This is achieved with an absorbent article that includes an absorbent body which comprises partially neutralised superabsorbent material, and a liquid-permeable fibrous top sheet which is bonded thermally to a porous liquid transfer sheet at discrete regions (e.g. punctiform/linear regions).

The invention thus relates to absorbent articles, such as diapers, sanitary napkins, incontinence protectors, wound dressings and the like, that comprises an absorbent body which is enclosed between a liquid-impermeable backing sheet and a material laminate comprised of a liquid-permeable, fibrous material sheet as a top sheet, and a liquid-permeable, porous and resilient material sheet as a liquid transfer sheet which lies proximal to the absorbent body, wherein the material laminate has a planar extension and a thickness direction perpendicular to the planar extension, wherein at least one of the material sheets comprises thermoplastic material, and wherein the two sheets of material are joined together through the medium of laminate bonding locations within which the thermoplastic material is caused to soften at least partially or to melt and therewith bind the two sheets together, and wherein the absorbent body includes partially neutralised superabsorbent, and wherein the sheet-joining regions or locations on the laminate extend in the thickness direction of the laminate through the top sheet and at least through a part of the liquid transfer sheet.

Plastic film is used as the top sheet in many types of absorbent articles. The benefit afforded by a fibre structure is that it reduces the risk of occlusion, which, in turn, reduces the risk of skin irritation. This is because a fibre structure is not as dense as film. A fibrous top sheet also presents a normally softer and smoother surface to the skin, therewith reducing the mechanical effect of the top sheet against the skin (e.g. chafing of the skin as the wearer moves).

The benefit afforded by a porous liquid acquisition layer or sheet between the liquid-permeable top sheet and the absorbent body, which is thermally bonded to the top sheet in discrete regions, is that the airiness of the fibrous outer sheet is retained to a better effect than when the whole surface of the top sheet or at least a large part of the surface thereof, is bonded to the surface of the liquid acquisition sheet. The discrete bonds also normally provide in the thickness direction of the laminate,a denser structure than in the non-bonded parts, which enables liquid to be guided more easily at the bond locations towards the inwardly lying porous liquid-acquisition structure.

Because the absorbent body includes partially neutralised superabsorbent material the pH will be lowered when said body is used, therewith counteracting undesired secondary effects, such as bad odours and skin irritation. This has a very good effect on the wearer in combination with the drier and softer top or outer sheet that faces the wearer in use. A typical degree of neutralisation is about 70%, although the degree of neutralisation will be lower in the case of the present invention.

The invention is particularly suitable for use in the prevention of diaper rash, among other things.

### BRIEF DESCRIPTION OF THE DRAWINGS

The invention will now be described in more detail with reference to the Figures of the accompanying drawings, in which
Fig. 1 illustrates from above a laminate included in an absorbent article according to the invention;
Fig. 2 is a sectional view through the laminate of Fig. 1, taken on the line II-II in said Figure;
Fig. 3 illustrates a first bonding pattern;
Fig. 4 illustrates a second bonding pattern;
Fig. 5 illustrates a third bonding pattern;
Fig. 6 illustrates a fourth bonding pattern;
Fig. 7 illustrates a fifth bonding pattern;
Fig. 8 illustrates a first embodiment of the invention in the form of an incontinence protector;
Fig. 9 is a diagrammatic illustration of the production of ammonia in a reference product as compared with a reference product 4; and
Fig. 10 is a diagrammatic illustration of the skin surface pH when using a test product that includes a conventional absorbent body as compared with the use of a corresponding test product 4.

### DESCRIPTION OF EMBODIMENTS

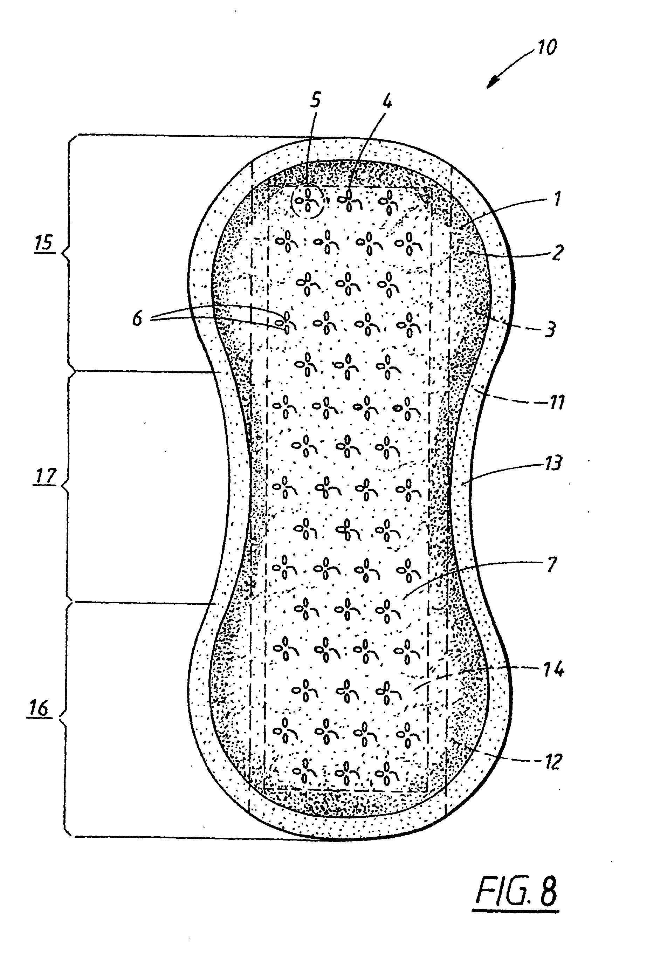

The invention relates to absorbent articles, such as diapers, sanitary napkins, incontinence protectors, wound dressings and the like. Fig. 8 shows by way of example an incontinence protector that includes an absorbent body or pad 12 enclosed between a liquid-impermeable backing sheet 11 and a material laminate 1 that comprises a liquid-permeable fibrous material sheet 2 as a top sheet, and a liquid-permeable porous, resilient material sheet 3 as a liquid transfer sheet 3. The liquid transfer sheet 3 faces towards the absorbent body 12 and the material laminate 1 has a planar extension and a thickness direction perpendicular to said planar extension. At least one of the material sheets 2, 3 includes thermoplastic material and the two sheets 2, 3 are bonded together through the medium of bonding regions 4 on the laminate 1, wherewith the thermoplastic material in said regions is caused to soften at least partially or to melt and thereby bond together the two sheets of material 2, 3. The absorbent body includes partially neutralised superabsorbent. The laminate bonding regions extend in the thickness direction of the laminate 1 through the top sheet 2 and at least partially through the liquid transfer sheet 3.

The bonding regions on the laminate 1 are disposed in two or more groups 5, with at least two bonding locations 4 in each group 5, wherewith the greatest distance between two mutually adjacent bonding locations 4 in a given group is shorter than the shortest distance between each group 5 and its nearest neighbouring group 5, said laminate 1 thereby having between the bonding locations 4 in each bonding group 5 bond-free regions 6 that have a higher density than those bond-free regions 9 in the laminate that are situated between said bonding groups 5.

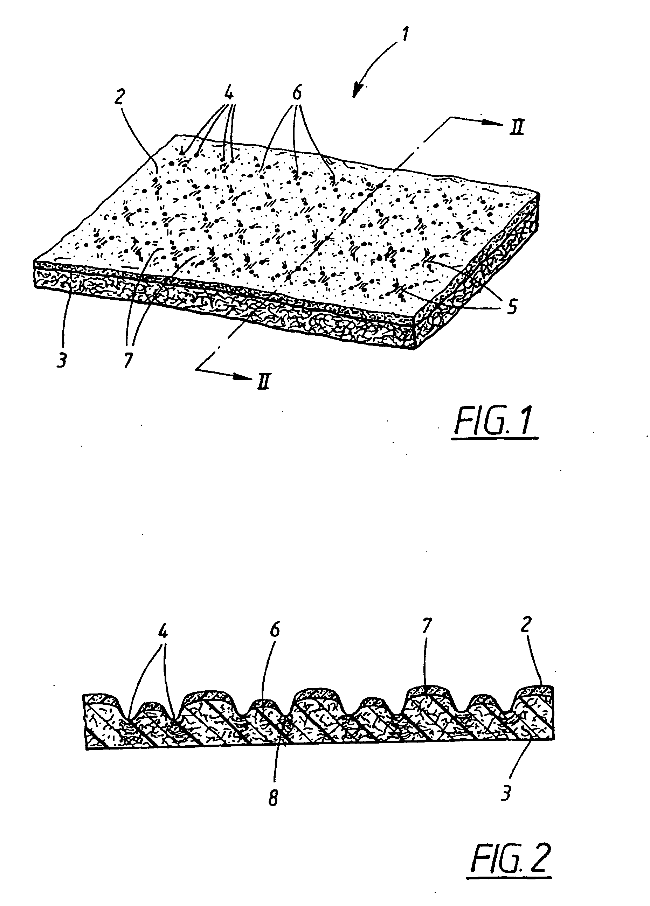

The laminate is described in more detail with reference to Figs. 1-7. The laminate 1 illustrated in Figs. 1 and 2 includes a first material sheet 2, the top sheet 2, and a second material sheet 3, the liquid transfer sheet 3. The first material sheet 2 is conveniently comprised of a relatively thin nonwoven material.

Nonwoven material can be produced in many ways, for instance by carding or spinning a fibrous mat and then bonding the mat. A melt-blow technique can be used to deposit short fibres in the form of a fibre mat. The fibres in nonwoven material can be bonded in any one of a number of different ways. For instance, different types of binder can be used. Furthermore, hot-melt components present in the material can be used to effect bonding by ultrasound or by applying heat. Other bonding methods are needling and hydro-entangling. A combination of different bonding methods may also be used.

When the laminate is used as a liquid-permeable top material on an absorbent article, the first material sheet 2, the top sheet 2, is the sheet which is intended to lie proximal to the wearer of the article. It is therewith important that the first sheet has a smooth and soft surface against the wearer.

The second material sheet 3, the liquid transfer sheet 3, will preferably be thicker than the first material sheet 2, and is comprised of a porous, resilient fibre material having a thickness of from 0.5 to 4 mm. This second material sheet 3 serves as a liquid transfer sheet when the laminate is mounted on an absorbent article as a top sheet. The second material sheet 3 will therefore preferably be able to accommodate large volumes of liquid in a short space of time and to spread or disperse liquid in the plane of said material sheet, pass the liquid to an absorbent body disposed beneath the laminate 1, and also be able to store temporarily liquid that has not had time to be absorbed by the absorbent body. Materials that are particularly suitable for use in the liquid transfer sheet 3 are synthetic fibre wadding, carded bonded or non-bonded fibre layers, or bulky nonwoven material. One particular type of fibre material that can be used in this context is tow, by which is meant essentially parallel, long or infinite fibres or fibre filaments which exist in the form of layers or strings. Another suitable material in this context is porous hydrophilic foam material. The second material sheet may also consist of two or more layers of different material or of one and the same type of material.

By way of a non-limiting example of a laminate that forms the top sheet of an inventive absorbent article can be mentioned a composite nonwoven material comprised of a first material sheet 2 of nonwoven synthetic fibre material that has a weight per unit area of between 10 and 50 g/m², and a second material sheet 3 comprised of synthetic fibre wadding and having a weight per unit area of between 20 and 100 g/m². At least the first sheet 2, and preferably both sheets 2, 3, will include thermoplastic material. Suitable thermoplastic material is polyester, such as polyethylene and polypropylene, and polyamides, polyester and the like. Other types of bicomponent fibres may be used.

The two sheets 2, 3 are joined together through a large number of bonding locations 4. The bonding locations 4 are essentially punctiform and have been formed by compressing the laminate 1 and supplying energy thereto simultaneously. The thermoplastic material has therewith been softened or melted at the bonding locations 4 such as to bond together the two sheets 2, 3 of the laminate 1. The first and the second sheets 2, 3 are suitably bonded together by thermal bonds or by ultrasound bonding in the form of welding, for instance. The welding pattern formed herewith has a three-dimensional structure.

The bonding locations 4 are disposed in groups 5 with four bonding locations 4 in each group 5. The four bonds are placed so as to form the corners of a square. The distance between the bonding locations 4 in each group is shorter than the distance between adjacent groups 5. The distance between the bonding locations within the groups 5 themselves is determined as the nearest distance between mutually adjacent bonding locations 4. Correspondingly, the distance between the groups 5 is determined as the nearest distance between mutually adjacent groups 5. The distances are measured from the edges of the bonding locations 4 in both cases. The shortest distance x between adjacent groups, measured between the bonding locations 4 closest together in respective groups 5, is suitably 2-6 mm, and the greatest distance y between mutually adjacent binding locations 4 in the groups is suitably 0.5-1 mm. The first-mentioned distance x is therewith at least roughly twice as large as the last-mentioned distance y. The x/y ratio between the distances x and y is from 2/1 to 12/1.

As the molten or softened thermoplastic material in the laminate 1 cools, it will solidify and function as a laminate bonding agent. In addition to this bonding of the two sheet 2, 3, the porous structure in the sheets 2, 3 remains compact or dense. Most pronounced is the densification at the actual bonding locations 4. The particular positioning of the bonding locations 4 means that the bonded laminate 1 will exhibit square areas 6 that are bordered by the bonding locations 4 in the groups 5, and will be denser in these regions than in the regions 7 between the groups 5.

The sheets forming the laminate 1 shown in Figs. 1 and 2 are bonded together by forming through-penetrating holes 8 in the top sheet 2 at the binding locations 4. In addition, the material situated within and nearest to the bonding locations 4 is greatly densified and has finer capillaries than the surrounding material. This enhances the ability of the regions in which the bonds are located to allow liquid to pass from the top sheet 2 to the liquid transfer sheet 3.

Although the laminate 1 is shown to include through-penetrating holes 8 in the first sheet 2, the top sheet 2, it will be understood that this is not a necessary feature of the invention. Thus, the invention also includes laminates in which the bonding locations 4 exhibit a surface of a more or less liquid-impermeable nature, and a laminate that includes both through-penetrating holes and liquid-impermeable bonds. Bonding locations of low liquid permeability or liquid impermeability are obtained, for instance, when the laminate includes a high proportion of thermoplastic material which is melted and then allowed to solidify into a film-like surface. Although the actual bonding locations 4 are practically totally impervious to liquid, the compacted fibre structure created around the bonding locations 4 in conjunction with the bonding compression that takes place nearest each bonding location 4 still has a very high liquid transfer capacity.

Furthermore, the densified regions 6 inwardly of the bonding locations 4 in each group 5 of bonding locations form zones of elevated liquid transfer capacity. Because the distance between the bonding locations 4 in each group 5 is relatively small, preferably from 0.5 mm to 1 mm, compression of the material in the bonding locations 4 will also affect the area 6 inwardly of said bonding locations 4 such as to obtain a denser structure. Thus, the size of the capillaries in the densified region 6 delimited by the bonding locations 4 is, on average, smaller than the size of the capillaries in those regions of the laminate 1 that are located between the groups 5 of bonding locations 4. The laminate 1 will thus have a relatively high liquid transfer capacity in relation to the combined surface area of the bonding locations 4. The combined bonded surface area will preferably be from 3 to 11% of the total surface area. The surprisingly good liquid transport and liquid transfer capacity of the laminate is not due solely to the bonding locations 4 themselves and to the regions or areas situated immediately adjacent these locations and exhibiting an elevated liquid transfer capacity, but is also due to those areas or regions located between the bonding locations 4 in a group 5 which also contribute to the improved liquid transfer capacity.

The invention thus enables regions of greater density, and therewith enhanced liquid transport capacity, to be created while still obtaining a high bulk laminate 1 which is soft and pliable. This results in a drier surface against the wearer's skin and in a product that has a lower pH, due to the absorbent body including partially neutralised superabsorbent. The risk of undesired side effects, such as bad odours and skin irritation, is also reduced.

All use of products that are applied to skin can lead to undesired side effects. These side effects can be caused by occlusion, the presence of moisture, and factors of a mechanical, a microbial and an enzymatic nature. Such use can also cause skin irritation, primary or secondary skin infections and generate undesirable odours. An increase in pH is a normal occurrence when absorbent products are worn against the skin. However, several undesired side effects can occur as a result of or in conjunction with an increase in pH. Irritative contact dermatitis, which is shown to have a relationship with a surface pH of the skin, is one example of such undesired side effects.

Another example of undesired side effects is that certain bacteria, such as Proteus, are able to metabolise the substances in urine and other body fluids and give rise to odorous substances, such as ammonia and amines, which also cause an increase in pH. At high pH values, the equilibrium of many odorous substances is displaced so as to generate more volatile components and are therefore more malodorous than at low pH values.

An environment such as that found in an absorbent article in which moisture, nutrients and heat, among other things, are available also favours the growth of micro-organisms. High bacteria numbers constitute an infection risk. A high bacterial presence also means a greater risk that embarrassing odours will be produced by the different substances that form as a result of the biological or chemical degradation of body fluid constituents, such as the constituents of urine and menstrual fluid. The activity of micro-organisms is greatly dependent on pH and decreases with falling pH values.

The use of a partially neutralised superabsorbent material in the absorbent structure according to the invention results in a decrease in pH. The aforesaid undesired side effects are thus reduced in the case of an inventive absorbent structure.

Partially neutralised superabsorbent material is used in absorbent articles described in Swedish Patent Application SE 9702298-2. A reduced pH value is obtained as a result of including in the material a pH-controlling substance in the form of a partially neutralised superabsorbent material. It has been found that a pronounced growth-inhibiting effect is obtained with respect to undesired strains of micro-organisms and the occurrence of undesired side effects that can result from the use of the article is reduced, when the pH of the absorbent article lies in the range of 3.5-4.9 or preferably 4.1-4.7 after wetting of the article.

A suitable, partially neutralised superabsorbent material may be comprised of a cross-linked polyacrylate of the kind described in European Patent Specification EP 0 391 108, Casella AG. Superabsorbent materials other than the aforesaid material that have corresponding properties may alternatively be used.

Examples of relationships between degrees of neutralisation and pH values of the superabsorbent material will be evident from the following text. The information listed below has been taken from Swedish Patent Application SE 9702298-2.

| Degree of neutralisation % | pH |
|---|---|
| 18 | 4.0 |
| 25 | 4.3 |
| 30 | 4.5 |
| 35 | 4.7 |
| 45 | 5.0 |
| 60 | 5.5 |

It will be evident from the table that the degree of neutralisation should normally be lower than 45% and preferably lower than 35%. The degree of neutralisation, however, should preferably be higher than about 20%. These degrees of neutralisation are also appropriate with respect to the present invention.

At those degrees of neutralisation used in the absorbent structure of an absorbent article in accordance with the invention, there is obtained an acid environment after the structure has been wetted when worn against the skin, therewith inhibiting the growth of micro-organisms and avoiding offensive odours and skin irritation.

After being wetted, the absorbent body of the inventive absorbent article will have a pH in the range of 3.5-4.9, preferably in the range of 4.1-4.7.

Another benefit afforded by the invention is that the occurrence, e.g., of offensive odours and skin complaints as a result of wearing the absorbent article against the skin are avoided. The growth-inhibiting effect is based on the fact that many micro-organisms have an activity which is strongly pH-dependent and decreases with decreasing pH values. Enzymes such as lipases and proteases also have an activity which is strongly pH-dependent and which decreases with decreasing pH values. Thus, a reduction in pH results in a reduction in the activity of the majority of micro-organisms and also in a reduction in enzyme activity, therewith providing a reduction in negative skin affects.

The following examples have been taken from SE 9702298-2 to illustrate the effect of absorbent articles that have an absorbent body which includes a partially neutralised superabsorbent material. The absorbent body also includes a cellulose pulp having a pH of 2.5-8.5.

An absorbent body that contains absorbent material and absorbed liquid is by nature a heterogeneous system from a pH aspect. The system may include superabsorbent material, fibres, and liquid that contains several types of ions. In order to obtain reproducible pH values, it is necessary to take measurements at several places in the sample body and to calculate the mean value of such measurements.

### DESCRIPTION OF EXAMPLES:

The following examples are intended to illustrate more closely the effect in absorbent articles that have an absorbent body which includes a combination of partially neutralised superabsorbent material and cellulose pulp having a pH of 2.5-8.5. Comparisons are made with conventional material of a corresponding type.

### TEST LIQUIDS:

### Test liquid 1

0.9% sodium chloride solution

### Test liquid 2

Synthetic urine according to the description of, *inter alia,* EP 0 565 606, such urine being obtainable from Jayco Pharmaceuticals Co., Pennsylvania. The urine has a composition of 2 g/l KCl; 2 g/l Na₂SO₄; 0.85 g/l (NH₄)H₂PO₄; 0.15 g/l (NH₄)₂HPO₄; 0.19 g/l CaCl₂ and 0.23 g/l MgCl₂. This mixture has a pH of 6.0-6.4.

### Test liquid 3

Synthetic urine containing the following substances: KCl, NaCl, MgSO₄, KH₂PO₄, Na₂HPO₄, NH₂CONH₂. This mixture has a pH of 6.0-6.5.

### Test liquid 4

Sterile synthetic urine to which a micro-organism growth medium has been added. The synthetic urine includes monovalent and divalent cations and anions and urea and was prepared in accordance with instructions given in Geigy, Scientific Tables, Vol. 2, 8th Ed., 1981, page 53. The micro-organism growth medium was based on data relating to Hook media and FSA media for enterobacteria. This mixture had a pH of 6.6.

### TEST METHODS:

### Method 1, the manufacture of absorbent bodies for test purposes

Absorbent bodies were produced with the aid of slightly modified test body formers in accordance with SCAN C 33:80. Fluff pulp and superabsorbent material of a desired kind were weighed and a uniform mixture of fluff pulp and superabsorbent material then passed in an air stream at a subpressure of about 85 mbar through a pipe having a diameter of 5 cm and provided with a bottom-carried metal net and a thin tissue placed on said net. The mixture of fluff pulp and superabsorbent material was collected on the tissue disposed on the metal net and thereafter formed the absorbent body. The absorbent body was weighed and then compressed to a bulk density of 6-12 cm³/g. A number of absorbent bodies designated Reference product 1, Reference product 2, test product 1, test product 2, test product 3, test product 4, and so on, of different compositions were then produced as described below. The quantity of absorbent material in the single core and in the double core absorbent bodies was adapted so that the single core bodies and the two core bodies had roughly the same absorption capacity.

### Method 2, measuring pH in the cellulose pulp

The pH of the cellulose pulp in the various test products was measured by determining the pH of a water extract from the pulp in accordance with SCAN P 14:65. 1.0 g of air-dry cellulose pulp was placed in a 100 ml glass beaker and 20 ml of distilled water were added. After stirring the mixture a further 50 ml of distilled water were added and the mixture then stirred for about 30 s. And allowed to stand for one hour. The liquid was then poured away and the pH measured with a glass electrode at 20-30°C. Two samples were made and the mean value calculated.

### Method 3, measuring the pH of an absorbent body

An absorbent body having a diameter of about 50 mm was produced in accordance with method 1. A certain amount of Test liquid 1, 2 and 3 was added, 10 ml to a single core absorbent body and 20 ml to a double core absorbent body. The absorbent body was then allowed to swell for 30 min., after which the pH of the absorbent body was measured with the aid of a surface electrode, flat bottom Metrohm pH-metre, Beckman ⌀12 or ⌀72. Parallel measurements were made on at least two different absorbent bodies. The pH was measured at 10 points on each absorbent body and the mean value then calculated.

### Method 4, measuring bacteria inhibition in absorbent bodies

Absorbent bodies were prepared in accordance with Method 1. Both single core and double core absorbent bodies were prepared. Test liquid 4 was prepared. Respective bacteria suspensions of Escherichia coli (E.c.), Proteus mirabilis (P.m.), Enterococcus faecalis (E.F.) were cultivated in nutrient broth at 30°C overnight. The graft cultures were diluted and the bacteria content calculated. The cultures were mixed in different proportions such that the final culture mix contained about 10⁴ organisms per ml of Test liquid 4. The Test liquid 4 was poured into a sterile sputum jar measuring 70.5 x 52 mm, and having a volume of 100 ml, and the absorbent body was placed upside down in the jar and allowed to absorb liquid over a period of 5 min., whereafter the jar was turned and incubated at 35°C for 0;6 and 12 hours respectively and the bacteria value in the absorbent body then determined. TGE agar was the nutrient used in measuring the total number of bacteria present, and Drigalski agar and Slanetz Bartley agar were used for specific measurement of Escherichia coli and Proteus mirabilis and Enterococcus faecalis respectively.

### Method 5, measuring the ammonia content

Single core absorbent bodies were prepared in accordance with Method 1. Test liquid and micro-organisms were added in accordance with Method 5, whereafter the jars were incubated at 35°C for 0.3, 6 and 12 hours respectively, whereafter samples were taken from the jars with the aid of a hand pump and so-called Dräger tubes. The ammonia content was then read-off as a colour indication along a scale graduated either in ppm or in percent by volume.

### Method 6, measuring the surface pH of the skin

Sample products were produced by coating the rear side of absorbent bodies according to reference 3 and Test 4 respectively with a coating of polyethylene that had a weight per unit area of about 25 g/m², and the front side of said bodies with polypropylene nonwoven coating that had a weight per unit area of about 20 g/m². Test liquid 3 was applied to the front side of the test product and absorbed therein. The resultant test products were placed on the forearms of test persons and allowed to remain there for 24 h. The procedure was repeated two times. The surface pH of the skin was measured at the place of contact prior to applying the test products, and after 24, 48 and 72 h. using a Courage + Khazaka skin pH meter that had a flat bottom Mettler-Toledo glass electrode 403/120.

### TEST PRODUCTS:

### Reference product 1:

A single core absorbent body having the total weight of 1 gram produced from a conventional superabsorbent material and a conventional chemithermomechanical cellulose pulp in a ratio of 15/85 weight-%.

### Test product 1:

A single core absorbent body having a total weight of 1 gram and produced from a partially neutralised superabsorbent material having a pH = 4.2 in accordance with the invention and a chemithermomechanical cellulose pulp having pH = 5.8, in a ratio of 15/85 weight-%.

### Test product 2:

A single core absorbent body having a total weight of 1 gram and produced from a partially neutralised superabsorbent material having pH = 4.2 in accordance with the invention and a chemithermomechanical cellulose pulp having pH = 3.7, in a ratio of 15/85 weight-%.

### Reference product 2:

A two core absorbent body. The upper core (UC) has a total weight of 1.2 gram and is produced from a conventional superabsorbent material and a conventional chemithermomechanical pulp in a ratio of 12/88%. The lower core (LC) has a total weight of 1.1 gram and is produced from a conventional superabsorbent material and a conventional chemical pulp in a ratio of 12/88 weight-%.

### Test product 3:

A two core absorbent body. The upper core (UC) had a total weight of 1.3 gram and was produced from a partially neutralised superabsorbent material having pH = 4.5 in accordance with the invention, and a chemithermomechanical pulp having pH 5.8, in a ratio of 15/85%. The lower core (LC) had a total weight of 1.2 gram and was produced from a partially neutralised superabsorbent material having pH = 4.5 in accordance with the invention, and a chemical pulp having pH = 6.3, in a ratio of 15/85 weight-%.

### Reference product 3:

A single core absorbent body has a total weight of 1 gram and is produced from conventional superabsorbent material and a conventional chemical cellulose pulp in a ratio of 15/85 weight-%.

### Test product 4:

A single core absorbent body having a total weight of 1 gram and produced from a partially neutralised superabsorbent material having pH = 4.2 according to the invention, and a conventional chemical cellulose pulp, in a ratio of 15/85 weight-%.

### Reference product 4:

A single core absorbent body having a total weight of 1 gram and produced from a conventional superabsorbent material and a chemithermomechanical cellulose pulp having pH = 6.7, in a ratio of 15/85 weight-%.

### Test product 5:

A single core absorbent body having a total weight of 1 gram and produced from a partially neutralised superabsorbent material having pH = 4.2 in accordance with the invention, and a chemithermomechanical cellulose pulp having pH = 6.7, in a ratio of 15/85 weight-%.

### Test product 6:

A two core absorbent body. The upper core (UC) has a total weight of 1.3 gram and is produced from a partially neutralised superabsorbent material having pH = 4.6 in accordance with the invention and a chemithermomechanical pulp having pH = 5.8, in a ratio of 15/85%. The lower core (LC) has a total weight of 1.2 gram and is produced from a partially neutralised superabsorbent material having pH = 4.6 in accordance with the invention and a chemical pulp having a pH = 6.3, in a ratio of 15/85 weight-%.

### TEST RESULTS:

### Example 1

As will be evident from Table 1, the growth of micro-organisms was good in a single core conventional absorbent body according to Reference product 1. Bacteria growth was measured in accordance with Method 4.

**Table 1:**

| Time | Esherichia coli | Proteus mirabilis | Enterococcus faecalis |
|---|---|---|---|
| 0 h | 3.3 | 3.1 | 3.7 |
| 6 h | 7.0 | 6.4 | 7.1 |
| 12 h | 9.2 | 9.1 | 8.3 |

### Example 2

It will be evident from Table 2 that the inhibition of the growth of micro-organisms was good in a single core absorbent body according to Test product 1. Bacteria inhibition was measured in accordance with Method 4.

**Table 2:**

| Time | Esherichia coli | Proteus mirabilis | Enterococcus faecalis |
|---|---|---|---|
| 0 h | 3.2 | 3.3 | 3.4 |
| 6 h | 5.5 | 3.2 | 4.8 |
| 12 h | 7.3 | 4.0 | 6.1 |

### Example 3

It will be evident from Table 3 that inhibition of the growth of micro-organisms was good in a single core absorbent body according to Test product 2. The measurements were carried out in accordance with Method 4.

**Table 3:**

| Time | Esherichia coli | Proteus mirabilis | Enterococcus faecalis |
|---|---|---|---|
| 0 h | 3.4 | 3.3 | 3.5 |
| 6 h | 3.2 | 2.6 | 3.6 |
| 12 h | 2.8 | 2.0 | 3.5 |

### Example 4

It will be evident from Table 4 that the growth of micro-organisms was good in a two core conventional absorbent body according to Reference product 2. The measurements were carried out according to Method 4.

**Table 4:**

| Time | Esherichia coli | | Proteus mirabilis | | Enterococcus faecalis | |
|---|---|---|---|---|---|---|
| | UC* | LC** | UC* | LC** | UC* | LC** |
| | | | | | | |
| 0 h | 3.4 | 3.4 | 3.4 | 3.4 | 3.4 | 3.4 |
| 6 h | 6.8 | 7.0 | 6.6 | 6.7 | 6.7 | 6.2 |
| 12 h | 9.0 | 9.0 | 9.1 | 9.0 | 8.0 | 7.8 |

| | | | | | | |
|---|---|---|---|---|---|---|
| UC* = upper core, | | | | | | |
| **LC = lower core | | | | | | |

### Example 5

It will be evident from Table 5 that the inhibition of the growth of micro-organisms was good in a two core absorbent body according to Test product 3. The measurements were carried out according to Method 4.

**Table 5:**

| Time | Esherichia coli | | Proteus mirabilis | | Enterococcus faecalis | |
|---|---|---|---|---|---|---|
| | UC* | LC** | UC* | LC** | UC* | LC** |
| 0 h | 3.4 | 3.4 | 3.4 | 3.4 | 3.4 | 3.4 |
| 6 h | 5.1 | 5.6 | 3.3 | 4.2 | 4.4 | 4.5 |
| 12 h | 7.3 | 7.4 | 4.0 | 4.0 | 5.9 | 4.8 |

| | | | | | | |
|---|---|---|---|---|---|---|
| UC* = upper core, | | | | | | |
| **LC = lower core | | | | | | |

### Example 6

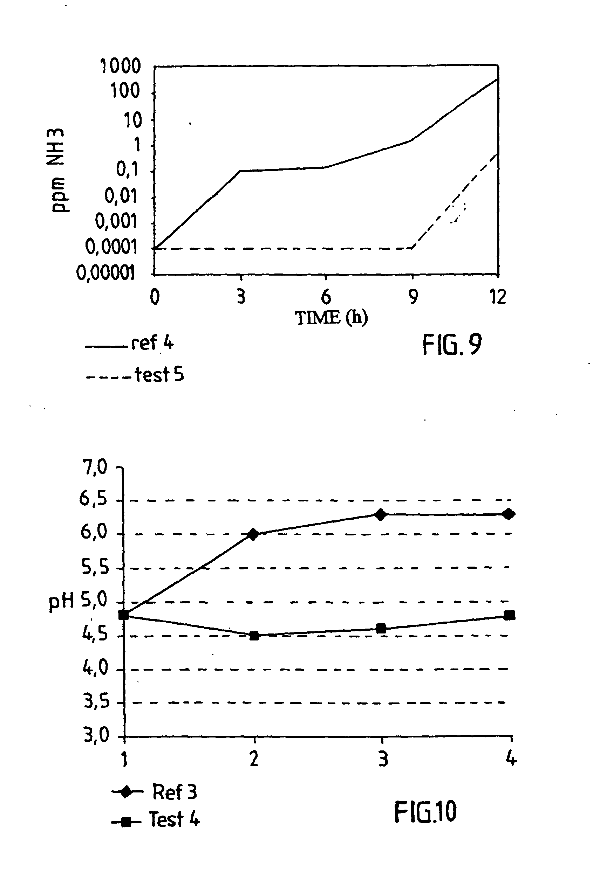

It will be evident from Fig. 9 that the production of ammonia was delayed effectively in a single core absorbent body according to Test product 5, in comparison with a single core conventional absorbent body according to Reference product 4. The measurements were carried out according to Method 5.

### Example 7

As will be evident from Fig. 10, after having used a sample product that included an absorbent body according to Test product 4 for a given period of time, the surface pH of the skin established itself at a lower level than in the case of a corresponding sample product containing a conventional superabsorbent material according to Reference product 3, after adding Test liquid 3. The measurements were carried out according to Method 6.

### Example 8

As will be evident from Table 6, the pH measured in a single core absorbent body according to Test product 1 lay within the active pH range of 3.5-4.9 after having added test liquid. The measurements were carried out according to Method 3.

**Table 6:**

| | Test liquid 1 | Test liquid 2 | Test liquid 3 |
|---|---|---|---|
| pH | 4.29 | 4.42 | 4.54 |

### Example 9

As will be seen from Table 7, after having added test liquid the pH measured in a two core absorbent body according to Test product 6 lay within the active pH range of 3.5-4.9. The measurements were carried out according to method 3.

**Table 7:**

| | Test liquid 1 | Test liquid 2 | Test liquid 3 |
|---|---|---|---|
| pH UC* | 4.72 | 4.83 | 4.80 |
| pH LC** | 4.75 | 4.73 | 4.73 |

| | | | |
|---|---|---|---|
| UC* = upper core, | | | |
| **LC = lower core | | | |

A lower pH thus has a good effect with respect to inhibiting the growth of micro-organisms. When a partially neutralised superabsorbent material is used together with the aforedescribed laminate in an absorbent article, further benefits with respect to skin irritation and odour are obtained. The described laminate also presents a drier surface to the skin of the wearer, which also has a good effect with respect to irritation of the skin. As will clearly be seen from Fig. 2 for instance, the weld pattern in the laminate of the inventive article has a three-dimensional structure. This means that less material will lie directly against the wearer's skin, therewith providing a degree of freeness between surface material and the wearer's skin. This reduces the risk of skin irritation caused, for instance, by chafing and/or by the skin becoming moist as a result of occlusion (heat) and/or because some liquid remains on the top sheet in contact with the wearer's skin after a first wetting.

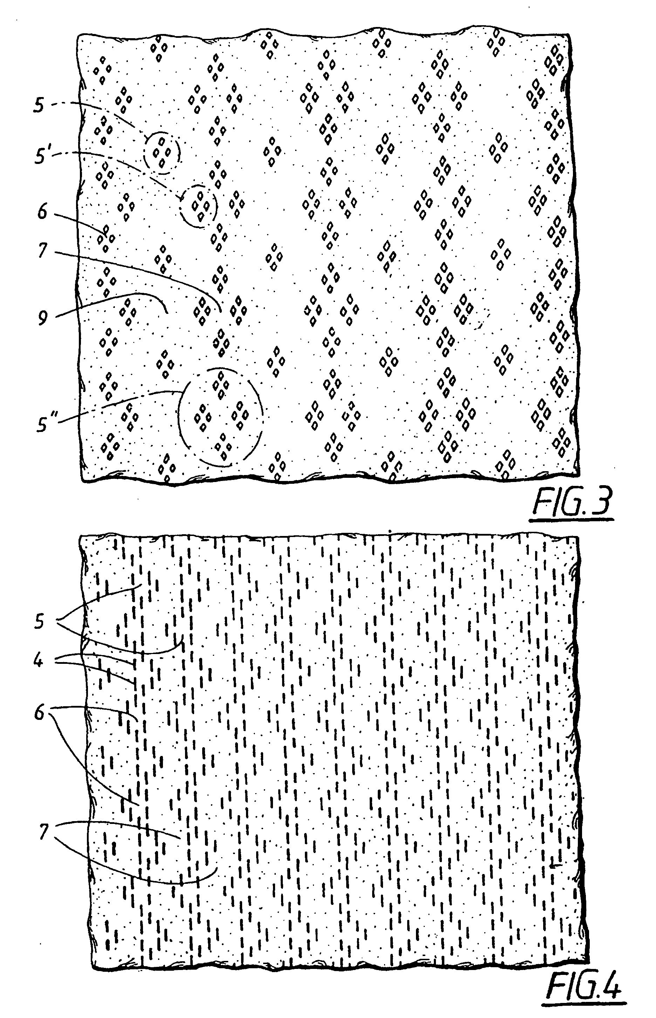

Described below are further embodiments of a laminate 1 used in accordance with the invention. Fig. 3 illustrates a bonding pattern in a laminate 1 whose uppermost sheet or layer lies proximal to the wearer of an inventive absorbent article. The binding pattern consists of rhombic bonding locations 4 disposed in groups 5', with four bonding locations 4 in each group 5'. The bonding pattern shown in Fig. 3 also includes superior group formations 5" comprising four groups 5' each having four bonding locations 4. Thus, three different types of regions 6, 7, 9 having mutually different densities in the material can be identified in the bonding pattern shown in Fig. 3. The densest material structure having the smallest pore size is found in the groups 5' that comprise four bonding locations 4. The less dense regions 7, which have a slightly large pore size, are found in the superior group formations 5" comprising groups 5' each having four bonding locations 4. Regions 9 of the lowest density are found between the superior group formations 5", and between the superior group formations 5" and single groups 5 of bonding locations 4 disposed between the superior group formations 5".

In the case of the Fig. 4 embodiment, the bonding locations 4 have the form of short (1-1.5 mm) dash-like bonds disposed in generally parallel stripe configurations 5 that are mutually spaced apart by a distance that is greater than the distance between the bonding locations 4 in said stripes. Located between the bonding locations 4 in respective stripes are densified regions 6 which have a smaller pore size than the regions 7 situated between said lines or stripes 5.

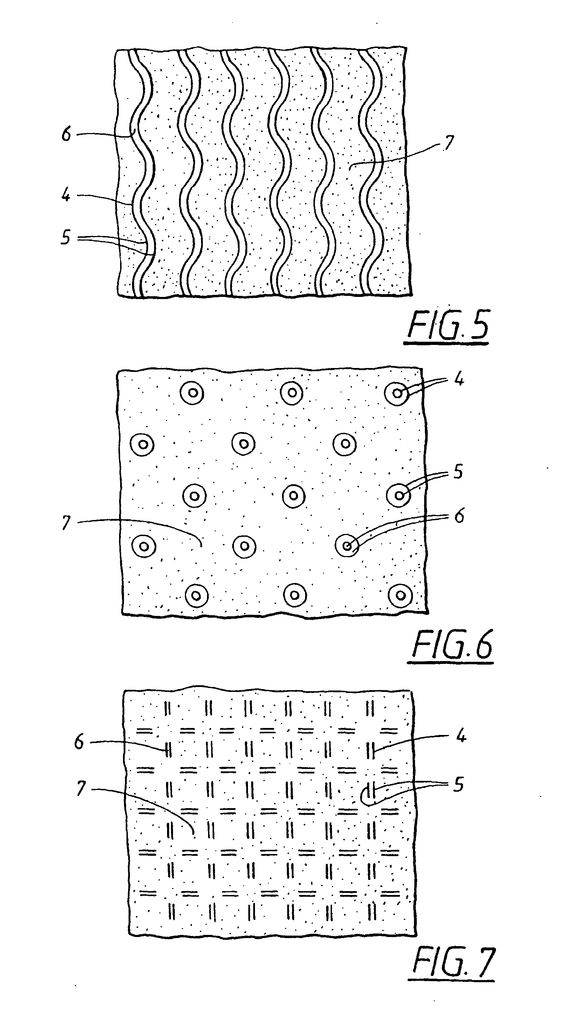

Figs. 5-7 illustrate further conceivable bonding patterns. The bonding pattern shown in Fig. 5 includes generally parallel, undulating pairs of bonding lines 4 where the distance between the lines 4 of each pair 5 exceeds the distance between the pairs 5 of bonding lines 4. Thus, there is obtained with the bonding pattern shown in Fig. 5 a laminate that includes densified liquid transfer regions between the bonding lines 4 of each pair and bulky, soft and airy spacing regions 7 between the bonding pairs 5.

One advantage that is afforded by arranging the bonding locations 4 in the form of stripes or lines is that a top material that includes such a bonding pattern will conduct liquid along the stripes or lines, and counteract the spread of liquid perpendicular to said stripes or lines. This facility can be used advantageously to reduce the risk of edge leakage in absorbent articles.

Fig. 6 illustrates a pattern which includes groups 5 each consisting of two bonding locations 4 in the form of concentric rings that delimit densified regions 6, while less dense regions 7 are found outside the outer ring of the ringshaped bonding locations 4.

Fig. 7 shows a pattern which comprises short, parallel dash-like lines 4 arranged in pairs at a given distance apart such as to form densified regions 6 between the dash-like lines 4 in each pair 5 and less dense regions between the pairs of dash-like lines 4.

Fig. 8 illustrates an embodiment of an inventive absorbent article in the form of an incontinence protector or napkin 10 that includes a laminate 1 which has a liquid permeable top sheet 2 and a liquid permeable liquid-transfer sheet 3. The article also includes a liquid-impermeable backing sheet 11 and an absorbent body or pad 12 enclosed between the top sheet 2 and the backing sheet 11. The top sheet 2 and the backing sheet 11 have a slightly greater extension than the absorbent body 12 and protrude slightly beyond the edges of said absorbent body. The top sheet 2 and the backing sheet 11 are joined together along their outwardly protruding parts 13, for instance by gluing or welding with heat or ultrasound.

The absorbent body 12 may be of any conventional kind. Examples of typical absorption material are cellulose fluff pulp, tissue, highly absorbent polymers (so-called superabsorbents), absorbent foam, absorbent nonwoven, and the like. It is also usual to construct absorbent bodies with layers of different materials that have different properties with respect to liquid acquisition capacity, liquid dispersing capacity and storage capacity. Such constructions are well known to the person skilled in this art and need not be described in detail here. The thin absorbent bodies or pads normal at present in, e.g., child diapers and incontinence protectors are often comprised of a compressed mixed or layered structure of cellulose fluff pulp and superabsorbent. According to the invention, the absorbent material is combined with partially neutralised superabsorbent in an absorbent body. As before mentioned, this results in an absorbent article that has a lower pH against the skin in use and that presents a dry surface to the skin. Irritation of the skin and the formation malodorous gases are counteracted by several factors, such as by inhibited growth of micro-organisms, less chafing of the skin, and less moisture in contact with the skin.

The incontinence protector or napkin 10 has an hourglass configuration, including broad end parts 15, 16 and a narrower crotch part 17 situated between said end parts 15, 16. The crotch part 17 is that part of the incontinence protector that is intended to lie between the thighs of the wearer in use and which functions as an acquisition surface for discharged body fluid.

As before mentioned, provided between the liquid-permeable top sheet 2 and the absorbent body 11 is a porous and resilient liquid transfer sheet 3, e.g. fibre wadding, a layer of porous foam or a layer of one of the materials mentioned above as being suitable for the second sheet in the laminate shown in Figs. 1 and 2. The liquid transfer sheet 3 receives the liquid that passes through the top sheet 2. Urination often involves the discharge of relatively large volumes of liquid over a short period of time. It is therefore important that the contact achieved between the liquid permeable top sheet and the inwardly lying liquid transfer sheet 3 is such that liquid is able to penetrate quickly into the liquid transfer sheet 3. Because the liquid transfer sheet has a high bulk density and a thickness of preferably 0.5-4 mm, the sheet 3 is able to function as a temporary liquid reservoir prior to being absorbed subsequently into the absorbent body 11.

In the illustrated embodiment, the liquid transfer sheet 3 is slightly narrower than the absorbent body 11 although extending along the full length of the incontinence protector. One advantage with this design is that it enables a saving in material consumption to be made. Naturally, a further saving can be made, by making the liquid transfer sheet 3 shorter than the length of the incontinence protector. For instance, one conceivable alternative in this respect is to place the liquid transfer sheet 3 solely at the crotch part 17 of said incontinence protector, since the major part of the body liquid, or fluid, to be absorbed by the incontinence protector can be expected to be discharged within this protector part 17.

Those liquid transfer sheets that are normally used are often very porous and therewith exhibit a relatively large effective mean size, which is often greater than the effective mean pore size of conventional liquid-permeable top sheet material. The effective medium pore size of a fibre material can be measured in accordance with a method described in EP-A-0 470 392. Since liquid strives to pass from coarse capillaries to finer capillaries and not vice versa, as a result of the capillary action, the liquid will tend to remain in the fibre network of the outer material instead of being drained away by the more porous liquid transfer sheet. Consequently, there is a danger that liquid will run on the surface of the outer sheet, or top sheet, and give rise to leakage. The liquid remaining in the fibre structure of the top sheet will also cause the surface of said sheet to be felt to be wet and therewith cause discomfort to the wearer.

By joining the liquid permeable top sheet 2 to the liquid transfer sheet 3 as described with reference to the laminate 1 shown in Figs. 1 and 2, the liquid transfer sheet 3 will be compressed at the bonding locations 4. Thus, the liquid transfer sheet 3 has a density gradient where the density increases in towards respective bonding locations 4. The liquid transfer sheet 3 will therefore present in a region around the bonding locations 4 a pore size gradient and an area in which the effective medium pore size is smaller than the mean pore size of the liquid permeable top sheet 2. By grouping the bonding locations 4 in accordance with the present invention, it is possible to increase that part of the laminate 1 surface at which the mean pore size of the liquid transfer sheet 3 is smaller than the mean pore size of the liquid permeable top sheet 2.

This enables the liquid transfer sheet 3 to drain liquid away from the top sheet 2 effectively. Because liquid is drained away from the top sheet 2 in the region surrounding respective bonding locations 4 and in the denser regions 6 that lie between the bonding locations 4 in each bonding location group 5, these regions will have a liquid deficit so as to achieve liquid equalisation with the surrounding regions. The top sheet 2 will therewith contain less liquid in total and will consequently be felt to be drier against the skin than would otherwise be the case. Since a lower pH is obtained when using the article, as a result of the partially neutralised superabsorbent material present in the absorbent body, the risk of, e.g., skin irritation is greatly reduced.

By arranging the bonding locations 4 in groups 5 with non-bonded densified regions 6 between said bonding locations 4, it is possible to achieve highly effective liquid transportation from the liquid permeable top sheet 2 to the liquid transfer sheet 3 with relatively few bonds. Further, non-bonded regions 7 are left between the groups 5, therewith giving an undulating or "bumpy" structure to the surface of the incontinence protector 10 that lies proximal to the wearer in use. These non-bonded regions 7 between the bonding groups 5 are bulky and soft, causing the laminate 1 to be airy and comfortable to wear and also effectively distancing said surface from the wearer's skin so that the skin will be kept dry even when the laminated is wetted.

In order to ensure that an effective liquid transfer is obtained between the liquid transfer sheet 3 and the absorbent body 11, the absorbent body will preferably have a greater affinity to liquid than the liquid transfer sheet 3. This can be achieved, for instance, by making the liquid transfer sheet 3 less hydrophilic than the absorbent body 11 and/or by giving the absorbent body 11 a finer capillary structure than the liquid transfer sheet 3.

## Claims

1. An absorbent article, such as a diaper, sanitary napkin, incontinence protector, wound dressing or the like, comprising an absorbent body (12) enclosed between a liquid-impermeable backing sheet (11) and a material laminate (1) in the form of a liquid permeable, fibrous sheet of material (2) forming a top sheet (2), and a liquid-permeable, porous and resilient sheet of material (3), forming a liquid transfer sheet (3) lying proximal to the absorbent body (12), wherein the laminate (1) has a planar extension and a thickness direction perpendicular to said planar extension, wherein at least one of the sheets (2, 3) includes thermoplastic material, and wherein the two sheets (2, 3) are joined together through the medium of bonding locations (4) on the laminate (1) within which the thermoplastic material is caused to at least partially soften or melt and thereby join together said two sheets (2, 3), **characterised in that** the absorbent body includes partially neutralised superabsorbent; and **in that** the sheet-joining regions of the laminate extend in the thickness direction of said laminate (1) through the top sheet (2) and at least partially through the liquid transfer sheet (3).

2. An absorbent article according to Claim 1, **characterised in that** the laminate bonding regions are disposed in two or more groups (5) where each group includes at least two bonding locations (4), wherein the largest relative distance between two mutually adjacent bonding locations (4) in a given group (5) is smaller than the smallest distance between a group (5) and its nearest neighbouring group (S), wherein the laminate (1) includes between the bonding locations (4) in each bonding group (5) first non-bonded laminate regions (6) that have a greater density than second non-bonded laminate regions (9) located between respective bonding groups (5).

3. An absorbent article according to Claim 1 or 2, **characterised in that** the superabsorbent has a degree of neutralisation such that the pH in the absorbent body of the article when wetted will lie in the range of 3.5-4.9, preferably 4.1-4.7.

4. An absorbent article according to Claim 2 or 3, **characterised in that** the laminate bonding locations (4) include punctiform bonds, linear bonds, rectangular bonds or circular bonds.

5. An absorbent article according to any one of the preceding Claims, **characterised in that** the top sheet (2) has through-penetrating holes within the bonding locations (4).

6. An absorbent article according to any one of the preceding Claims, **characterised in that** the top sheet (2) is comprised of a nonwoven material.

7. An absorbent article according to any one of the preceding Claims, **characterised in that** the top sheet (2) is comprised of a carded, thermobonded nonwoven material.

8. An absorbent article according to any one of the preceding Claims, **characterised in that** the liquid transfer sheet (3) is a fibre wadding sheet having a thickness of 0.5-4 mm.

9. An absorbent article according to any one of the preceding Claims, **characterised in that** the smallest distance x between two mutually adjacent groups (5) of bonding locations (4) is at least twice the size of the greatest distance y between two mutually adjacent bonding locations (4) in respective groups (5).

10. An absorbent article according to Claim 9, **characterised in that** the ratio of x/y between the distances x and y is from 2/1 to 12/1.

11. An absorbent article according to Claim 9 or 10, **characterised in that** x is 2-6 mm and y is 0.5-1 mm.

## Patentansprüche

1. Absorptionsartikel, wie z.B. Windel, Hygienebinde, Inkontinenzschutz, Wundverband oder ähnliches, mit einem Absorptionskörper (12), der zwischen einer flüssigkeitsundurchlässigen Stützlage (11) und einem Materiallaminat (1) in der Form eines flüssigkeitsdurchlässigen, faserigen Materialblattes (2), das eine Oberlage (2) bildet, und eines flüssigkeitsdurchlässigen, porösen und nachgiebigen Materialblattes (3), das eine Flüssigkeits-Übertragungslage (3) bildet, die proximal zu dem Absorptionskörper (12) liegt, eingeschlossen ist, wobei das Laminat (1) eine ebene Erstreckung und eine Dickenrichtung senkrecht zu der ebenen Erstreckung aufweist, wobei wenigstens eine der Lagen (2, 3) thermoplastisches Material aufweist, und wobei die beiden Lagen (2, 3) miteinander mittels Verbindungsstellen (4) an dem Laminat (1) verbunden sind, innerhalb welcher das thermoplastische Material veranlasst wird, wenigstens teilweise aufzuweichen oder zu schmelzen, und dadurch die beiden Lagen (2, 3) zu verbinden,
**dadurch gekennzeichnet, dass**
der Absorptionskörper ein teilweise neutralisiertes Superabsorbenzium aufweist, und dadurch, dass die blattverbindenden Bereiche des Laminats sich in der Dickenrichtung des Laminats (1) durch die Oberlage (2) und wenigstens teilweise durch die Flüssigkeits-Übertragungslage (3) erstrecken.

2. Absorptionsartikel nach Anspruch 1,
**dadurch gekennzeichnet, dass**
die Laminatverbindungsbereiche in zwei oder mehr Gruppen (5) angeordnet sind, wobei jede Gruppe wenigstens zwei Verbindungsstellen (4) aufweist, wobei der größte relative Abstand zwischen zwei zueinander benachbarten Verbindungsstellen (4) in einer gegebenen Gruppe (5) kleiner ist als der kleinste Abstand zwischen einer Gruppe (5) und ihrer am nächsten benachbarten Gruppe (5), wobei das Laminat (1) zwischen den Verbindungsstellen (4) in jeder Verbindungsgruppe (5) erste nicht verbundene Laminatbereiche (6) aufweist, die eine größere Dichte aufweisen als zweite, nicht verbundene Laminatbereiche (9), die zwischen jeweiligen Verbindungsgruppen (5) angeordnet sind.

3. Absorptionsartikel nach Anspruch 1 oder 2,
**dadurch gekennzeichnet, dass**
das Superabsorbenzium ein Neutralisationsausmaß derart aufweist, dass der pH in dem Absorptionskörper des Artikels, wenn benässt, in dem Bereich von 3,5-4,9, vorzugsweise 4,1-4,7 liegt.

4. Absorptionsartikel nach Anspruch 2 oder 3,
**dadurch gekennzeichnet, dass**
die Laminatverbindungsstellen (4) punktförmige Verbindungen, lineare Verbindungen, rechtwinklige Verbindungen oder kreisförmige Verbindungen aufweisen.

5. Absorptionsartikel nach einem der vorangehenden Ansprüche,
**dadurch gekennzeichnet, dass**
die Oberlage (2) durchdringende Öffnungen innerhalb der Verbindungsstellen (4) aufweist.

6. Absorptionsartikel nach einem der vorangehenden Ansprüche,
**dadurch gekennzeichnet, dass**
die Oberlage (2) aus Vliesmaterial besteht.

7. Absorptionsartikel nach einem der vorangehenden Ansprüche,
**dadurch gekennzeichnet, dass**
die Oberlage (2) aus einem kardierten, thermoverbundenen Vliesmaterial besteht.

8. Absorptionsartikel nach einem der vorangehenden Ansprüche,
**dadurch gekennzeichnet, dass**
die Flüssigkeits-Übertragungslage (3) eine Faserwattierungslage mit einer Dicke von 0,5-4 mm ist.

9. Absorptionsartikel nach einem der vorangehenden Ansprüche,
**dadurch gekennzeichnet, dass**
der kleinste Abstand x zwischen zwei zueinander benachbarten Gruppen (5) von Verbindungsstellen (4) wenigstens zweimal die Größe des größten Abstandes y zwischen zwei benachbarten Verbindungsstellen (4) in jeweiligen Gruppen (5) beträgt.

10. Absorptionsartikel nach Anspruch 9,
**dadurch gekennzeichnet, dass**
das Verhältnis von x/y zwischen den Abständen x und y von 2/1 bis 12/1 beträgt.

11. Absorptionsartikel nach Anspruch 9 oder 10,
**dadurch gekennzeichnet, dass**
x 2-6 mm und y 0,5-1 mm beträgt.

## Revendications

1. Article absorbant, tel qu'une couche-culotte, une serviette hygiénique, une protection contre l'incontinence, un pansement ou article similaire, comprenant un corps absorbant (12) enfermé entre une feuille de renfort (11) imperméable aux liquides et un matériau stratifié (1) sous la forme d'une feuille de matériau (2) fibreuse et perméable aux liquides formant une feuille supérieure (2), et une feuille de matériau (3) poreuse et élastique, perméable aux liquides, formant une feuille de transfert (3) de liquides se trouvant à proximité du corps absorbant (12), dans lequel le stratifié (1) a une étendue plane et une direction d'épaisseur perpendiculaire à ladite étendue plane, dans lequel au moins l'une des feuilles (2, 3) comprend un matériau thermoplastique, et dans lequel les deux feuilles (2, 3) sont liées ensemble par le biais d'emplacements de collage (4) sur le stratifié (1) dans lesquels on fait au moins partiellement ramollir ou fondre le matériau thermoplastique pour lier ensemble lesdites deux feuilles (2, 3), **caractérisé en ce que** le corps absorbant comprend un superabsorbant partiellement neutralisé, et **en ce que** les régions de liaison de feuille du stratifié s'étendent dans la direction de l'épaisseur dudit stratifié (1) à travers la feuille supérieure (2) et au moins partiellement à travers la feuille de transfert (3) de liquide.

2. Article absorbant selon la revendication 1, **caractérisé en ce que** les régions de collage du stratifié sont disposées en deux groupes (5) ou plus, chaque groupe comportant au moins deux emplacements de collage (4), où la plus grande distance relative entre deux emplacements de collage (4) mutuellement adjacents dans un groupe (5) donné est inférieure à la plus petite distance entre un groupe (5) et son groupe voisin (5) le plus proche, dans lequel le stratifié (1) comprend, entre les emplacements de collage (4) dans chaque groupe de collage (5), des premières régions de stratifié non collées (6) qui ont une plus grande densité que des deuxièmes régions de stratifié non collées (9) situées entre des groupes de collage (5) respectifs.

3. Article absorbant selon la revendication 1 ou 2, **caractérisé en ce que** le superabsorbant a un degré de neutralisation tel que le pH dans le corps absorbant de l'article, lorsqu'il est mouillé, se trouve dans l'intervalle de 3,5 à 4,9, de préférence de 4,1 à 4,7.

4. Article absorbant selon la revendication 2 ou 3, **caractérisé en ce que** les emplacements de collage (4) du stratifié comprennent des liens en forme de point, des liens linéaires, des liens rectangulaires ou des liens circulaires.

5. Article absorbant selon l'une quelconque des revendications précédentes, **caractérisé en ce que** la feuille supérieure (2) comporte des trous pénétrants à l'intérieur des emplacements de collage (4).

6. Article absorbant selon l'une quelconque des revendications précédentes, **caractérisé en ce que** la feuille supérieure (2) est constituée d'un matériau non tissé.

7. Article absorbant selon l'une quelconque des revendications précédentes, **caractérisé en ce que** la feuille supérieure (2) est constituée d'un matériau non tissé, cardé et thermocollé.

8. Article absorbant selon l'une quelconque des revendications précédentes, **caractérisé en ce que** la feuille de transfert (3) de liquides est une feuille de ouatage à fibres, ayant une épaisseur comprise dans l'intervalle de 0,5 à 4 mm.

9. Article absorbant selon l'une quelconque des revendications précédentes, **caractérisé en ce que** la plus petite distance x entre deux groupes (5) mutuellement adjacents d'emplacements de collage (4) fait au moins deux fois la taille de la plus grande distance y entre deux emplacements de collage (4) mutuellement adjacents dans les groupes (5) respectifs.

10. Article absorbant selon la revendication 9, **caractérisé en ce que** le rapport x/y entre les distances x et y va de 2/1 à 12/1.

11. Article absorbant selon la revendication 9 ou 10, **caractérisé en ce que** x est compris dans l'intervalle de 2 à 6 mm et y est compris dans l'intervalle de 0,5 à 1 mm.
